# EUROPEAN PATENT APPLICATION

(11) **EP 4 245 335 A1**
(43) Date of publication of application: **20.09.2023**
(21) Application number: 21891492.7
(22) Date of filing: 15.09.2021
(51) Int. Cl.: A61M 5/303, A61L 31/02, A61L 31/04

(54) **DEVICE ASSEMBLY FOR NEEDLELESS SYRINGE**

(30) Priority: 11.11.2020 JP 2020188349
(71) Applicant: Daicel Corporation, Osaka 530-0011 (JP)
(72) Inventor: IWAI, Yasunori, Tokyo 108-8230 (JP); SUZUKI, Takamasa, Tokyo 108-8230 (JP); YAMAMOTO, Yuzo, Tokyo 108-8230 (JP)
(74) Representative: Grünecker Patent- und Rechtsanwälte PartG mbB
(86) International application number: PCT/JP2021/033965
(87) International publication number: WO 2022/102244

(57) **Abstract**

A problem in the present disclosure is at least to provide a technique for intradermally injecting an injection objective substance, and the problem is solved by providing a device assembly for a needleless injector including a gas generating agent formed in a manner that a combustion rate thereof is lower than a combustion rate of an ignition agent and a combustion duration thereof is longer than a combustion duration of the ignition agent, the device assembly being configured to pressurize the injection objective substance in a manner that an ejection pressure of the injection objective substance, which is defined as a pressure of the injection objective substance ejected from an ejection port, increases to a first peak pressure after pressurization is started, then decreases to a pressure lower than the first peak pressure, and then increases again to a second peak pressure, the device assembly being configured to activate an igniter and cause the ejection pressure of the injection objective substance to reach the first peak pressure by a pressure of a combustion gas of the ignition agent, and configured to cause the ejection pressure of the injection objective substance to reach the second peak pressure by application of the pressure of the combustion gas of the gas generating agent to be combusted subsequently to the ignition agent, wherein the ignition agent and the gas generating agent generate a predetermined gas having a predetermined energy value in combustion.

## Description

### TECHNICAL FIELD

The present disclosure relates to a device assembly for a needleless injector.

### BACKGROUND ART

An injector can be exemplified as a device that administers a liquid chemical to a target region such as an organism. In recent years, a needleless injector that does not include an injection needle has been developed in view of handling facilitation, sanitation, and the like. In general, there has been implemented a needleless injector having a configuration in which a liquid chemical pressurized by a drive source such as compressed gas and a spring is ejected to a target region and the liquid chemical is administered to an inside of the target region through use of the kinetic energy of the liquid chemical. As another drive source, use of explosive combustion has been examined. As described above, the needleless injector does not have a mechanical configuration that is held in direct contact with the inside of the target region (for example, an injection needle), and hence convenience for a user is high. Meanwhile, due to absence of such mechanical configuration, it is not always easy to administer the liquid chemical to a desired part in the target region.

Here, Patent Document 1 discloses a technique of sending a liquid chemical into a desired depth of a skin structure body of an organism with a needleless injector. Specifically, the technique relates to pressurizing for ejection of an injection solution, with pressurizing control having a first pressurizing mode and a second pressurizing mode being performed. In the first pressurizing mode, after a pressure is raised to a first peak pressure, the pressure to the injection solution is lowered to a standby pressure, and thus a through-passage is formed in an injection target region. In the second pressurizing mode, pressurizing is performed to the injection solution having the standby pressure, the pressure of the injection solution is raised to a second peak pressure, and thus a predetermined injection amount is injected. By performing such pressurizing control, the behavior of the injection solution in the target region is controlled.

Additionally, Patent Document 2 discloses a technique of accurately adjusting a reaching depth of an ejected injection objective substance in the target region with the needleless injector that injects the injection objective substance to the target region without using an injection needle.

### CITATION LIST

### PATENT DOCUMENT

Patent Document 1: JP 2012-61269 A
Patent Document 2: JP 2019-5406 A

### SUMMARY OF INVENTION

### TECHNICAL PROBLEM

An object of the present disclosure is at least to provide a technique for intradermally injecting an injection objective substance.

### SOLUTION TO PROBLEM

In order to solve the above problem, the inventors of the present invention focused on an energy amount of gas generated in combustion of an ignition agent and a gas generating agent that are included in a device assembly for a needleless injector that intradermally injects an injection objective substance without using an injection needle. As a result, the inventors of the present invention have newly found that the ejected injection objective substance can be intradermally injected by setting the energy parameter within a predetermined range.

Specifically, as an embodiment of the present disclosure,
there can be provided a device assembly for a needleless injector configured to intradermally inject an injection objective substance without using an injection needle, the device assembly including
an enclosing unit configured to enclose the injection objective substance,
a drive unit including an igniter including an ignition agent and a gas generating agent disposed in a combustion chamber into which a combustion product generated by combustion of the ignition agent flows, the gas generating agent being configured to be ignited by the combustion product and thus to generate gas,
a pressurizing unit configured to pressurize, when the drive unit is driven, the injection objective substance enclosed in the enclosing unit, and
an ejection port through which the injection objective substance pressurized by the pressurizing unit is ejected to a target region,
wherein the gas generating agent is formed in a manner that a combustion rate of the gas generating agent is lower than a combustion rate of the ignition agent, and a combustion duration of the gas generating agent is longer than a combustion duration of the ignition agent,
the pressurizing unit is
configured to pressurize the injection objective substance in a manner that an ejection pressure of the injection objective substance increases to a first peak pressure after starting pressurization, then decreases to a pressure lower than the first peak pressure, and then increases again to a second peak pressure, the ejection pressure being defined as a pressure of the injection objective substance ejected from the ejection port,
the drive unit is
configured to activate the igniter and cause the ejection pressure of the injection objective substance to reach the first peak pressure by a pressure of a combustion gas of the ignition agent, and configured to cause the ejection pressure of the injection objective substance to reach the second peak pressure by application of the pressure of the combustion gas of the gas generating agent to be combusted subsequently to the ignition agent,
the ignition agent generates a combustion gas having a gas amount n1 (mol) and a temperature T1 (K) in combustion, and is defined in a manner that an energy value represented by n1 × T1 (mol · K) in the combustion gas of the ignition agent is from 1.0 mol · K to 2.7 mol · K, and
the gas generating agent generates a combustion gas having a gas amount n2 (mol) and a temperature T2 (K) in combustion, and is defined in a manner that an energy value represented by n2 × T2 (mol · K) in the combustion gas of the gas generating agent is from 3.7 mol · K to 9.2 mol · K.

In the device assembly for the needleless injector, the ignition agent may be any one of an explosive containing zirconium and potassium perchlorate, an explosive containing zirconium, tungsten, and potassium perchlorate, an explosive containing titanium hydride and potassium perchlorate, or an explosive containing titanium and potassium perchlorate, or an explosive containing a combination of a plurality the explosives among these explosives.

In the device assembly for the needleless injector, the gas generating agent may be a gas generating agent including any one of a single-base gas generating agent, a double-base gas generating agent, or a triple-base gas generating agent each containing nitrocellulose, or a gas generating agent including a combination of a plurality of gas generating agents among these gas generating agents.

The present disclosure can provide a needleless injector including the device assembly for the needleless injector described above and a control unit to which the device assembly for the needleless injector is attached, the control unit being configured to generate an activation signal for the igniter.

The present disclosure can also provide a method for intradermally injecting an injection objective substance by using the needleless injector.

### ADVANTAGEOUS EFFECTS OF INVENTION

Patent Document 2 discloses a technique for accurately adjusting a reaching depth of an ejected injection objective substance in a target region, but does not refer to any conditions for intradermal injection. On the other hand, according to the present disclosure, it is possible to provide at least a technique for intradermally injecting an injection objective substance.

There are a number of advantages due to being able to intradermally inject the injection objective substance. For example, dendritic cells (Langerhans cells) that recognize and phagocytose antigens, transmit information to T cells and the like, and activate lymphocytes are contained in the intradermal space. Thus, for example, a more effective antigen-antibody reaction can be induced by causing a vaccine to reach the intradermal space.

Further, pigment cells having a function of preventing influence of ultraviolet light emitted from an external environment are included in the intradermal space. As a result, for example, by causing a predetermined substance for whitening to reach the intradermal space, it is possible to take a measure for so-called whitening use.

### BRIEF DESCRIPTION OF DRAWINGS

FIG. 1 is a view illustrating a schematic configuration of an injector driven by an ignition agent.
FIG. 2A is a view illustrating a schematic configuration of a first sub-assembly that forms a device assembly incorporated in the injector illustrated in FIG. 1.
FIG. 2B is a view illustrating a schematic configuration of a second sub-assembly that forms the device assembly incorporated in the injector illustrated in FIG. 1.
FIG. 3 is a view illustrating a change in ejection pressure of an injection solution ejected by the injector illustrated in FIG. 1.
FIG. 4 is a view illustrating a structure body of the skin.

### DESCRIPTION OF EMBODIMENTS

Each of the configurations, combinations thereof, and the like in each embodiment are an example, and various additions, omissions, substitutions, and other changes may be made as appropriate without departing from the spirit of the present invention. The present disclosure is not limited by the embodiments and is limited only by the claims. Each aspect disclosed in the present specification can be combined with any other feature disclosed herein.

In the present disclosure, the expression "the injection objective substance reaches the intradermal space" indicates that the injection objective substance eventually reaches the intradermal space, and this can be determined by the formation of a water bubble (that is a state in which the skin surface is swollen by the injection solution) at the injection site after injection. When no water bubble is formed after injection, for example, it indicates that most of the injection solution has been delivered to the subcutaneous space of the skin or to the muscle, and in this case, it is not considered that "the injection objective substance has reached the intradermal space". In addition, for example, as will be described later, even when the injection objective substance once reaches the intradermal space or at a position deeper than the intradermal space but the injection objective substance is pushed back to the skin surface or the horny layer by restoration (elasticity) of the skin, and eventually, the injection objective substance does not reach the intradermal space, it is not determined that "the injection objective substance has reached the intradermal space ".

According to an embodiment of the present disclosure, a device assembly for a needleless injector configured to intradermally inject an injection objective substance without using an injection needle includes an enclosing unit configured to enclose the injection objective substance, a drive unit including an igniter including an ignition agent and a gas generating agent disposed in a combustion chamber into which a combustion product generated by combustion of the ignition agent flows, the gas generating agent being configured to be ignited by the combustion product and thus to generate gas, a pressurizing unit configured to pressurize the injection objective substance enclosed in the enclosing unit when the drive unit is driven, and an ejection port through which the injection objective substance pressurized by the pressurizing unit is ejected to a target region.

Further, the gas generating agent is formed in a manner that a combustion rate of the gas generating agent is lower than a combustion rate of the ignition agent and a combustion duration of the gas generating agent is longer than a combustion duration of the ignition agent.

Further, the pressurizing unit is configured to pressurize the injection objective substance in a manner that an ejection pressure of the injection objective substance increases to a first peak pressure after starting pressurization, then decreases to a pressure lower than the first peak pressure, and then increases again to a second peak pressure, the ejection pressure being defined as a pressure of the injection objective substance ejected from the ejection port, and
the drive unit is
configured to activate the igniter and cause the ejection pressure of the injection objective substance to reach the first peak pressure by a pressure of a combustion gas of the ignition agent, and configured to cause the ejection pressure of the injection objective substance to reach the second peak pressure by application of the pressure of the combustion gas of the gas generating agent to be combusted subsequently to the ignition agent.

Furthermore, the ignition agent generates a combustion gas having a gas amount n1 (mol) and a temperature T1 (K) in combustion, and is defined in a manner that an energy value represented by n1 × T1 (mol · K) in the combustion gas of the ignition agent is from 1.0 mol · K to 2.7 mol · K, and
the gas generating agent generates a combustion gas having a gas amount n2 (mol) and a temperature T2 (K) in combustion, and is defined in a manner that an energy value represented by n2 × T2 (mol · K) in the combustion gas of the gas generating agent is from 3.7 mol · K to 9.2 mol · K.

In both the ignition agent and the gas generating agent, an energy of each gas is expressed as nRT obtained by multiplying an amount n of gas generated and a temperature T of the gas by a gas constant R. Here, R becomes a gas constant of ideal gas as the gas temperature increases, and becomes a constant value. Thus, nT without R is an energy alternative value that characterizes an energy value, but is expressed as an energy value (or simply expressed as an energy) in the present disclosure.

The device assembly for the needleless injector according to the present embodiment can be configured as a cartridge configured to be attachable to and detachable from a housing of the needleless injector according to another embodiment, which will be described later. The housing includes a battery that supplies power to an igniter included in the drive unit of the device assembly. The housing is a control unit that can be repeatedly used as long as power that can be applied to the drive unit is left in the battery. For these details, description of the needleless injector according to another embodiment, which will be described later, will be cited.

In other words, a needleless injector according to another embodiment, which will be described later, is configured to include the device assembly for the needleless injector according to the present embodiment and the control unit to which the device assembly for the needleless injector is attached and that generates an activation signal for the igniter. In addition, the device assembly for the needleless injector in the needleless injector can be detachably attached to the control unit, and the device assembly for the injector can be configured as a cartridge that is disposable each time the injection objective substance is intradermally injected.

Another embodiment of the present disclosure is a needleless injector including a device assembly for a needleless injector and a control unit to which the device assembly for the needleless injector is attached and that generates an activation signal for the igniter.
That is, the needleless injector according to the present disclosure is
a needleless injector configured to intradermally inject an injection objective substance without using an injection needle, including
a device assembly for a needleless injector, and
a control unit to which the device assembly for the needleless injector is attached, the control unit being configured to generate an activation signal for the igniter,
wherein the device assembly for the needleless injector includes
an enclosing unit configured to enclose the injection objective substance,
a drive unit including an igniter including an ignition agent and a gas generating agent disposed in a combustion chamber into which a combustion product generated by combustion of the ignition agent flows, the gas generating agent being configured to be ignited by the combustion product and thus to generate gas,
a pressurizing unit configured to pressurize the injection objective substance enclosed in the enclosing unit when the drive unit is driven, and
an ejection port through which the injection objective substance pressurized by the pressurizing unit is ejected to a target region,
the gas generating agent is formed in a manner that a combustion rate of the gas generating agent is lower than a combustion rate of the ignition agent and a combustion duration of the gas generating agent is longer than a combustion duration of the ignition agent,
the pressurizing unit is
configured to pressurize the injection objective substance in a manner that an ejection pressure of the injection objective substance increases to a first peak pressure after starting pressurization, then decreases to a pressure lower than the first peak pressure, and then increases again to a second peak pressure, the ejection pressure being defined as a pressure of the injection objective substance ejected from the ejection port,
the drive unit is
configured to activate the igniter and cause the ejection pressure of the injection objective substance to reach the first peak pressure by a pressure of a combustion gas of the ignition agent, and configured to cause the ejection pressure of the injection objective substance to reach the second peak pressure by application of the pressure of the combustion gas of the gas generating agent to be combusted subsequently to the ignition agent,
the ignition agent generates a combustion gas having a gas amount n1 (mol) and a temperature T1 (K) in combustion, and is defined in a manner that an energy value represented by n1 × T1 (mol · K) in the combustion gas of the ignition agent is from 1.0 mol · K to 2.7 mol · K, and
the gas generating agent generates a combustion gas having a gas amount n2 (mol) and a temperature T2 (K) in combustion, and is defined in a manner that an energy value represented by n2 × T2 (mol · K) in the combustion gas of the gas generating agent is from 3.7 mol · K to 9.2 mol · K.

In the needleless injector according to the present embodiment, the pressurizing unit pressurizes the injection objective substance enclosed in the enclosing unit, and thus the injection objective substance is ejected to the target region. In the needleless injector according to the present embodiment, an energy used for pressurization is a combustion energy of the ignition agent (an energy of combustion gas of the ignition agent) ignited in association with activation of the igniter and a combustion energy of the gas generating agent (an energy of combustion gas of the gas generating agent) ignited by the combustion gas of the ignition agent.

Further, examples of the injection objective substance ejected from the needleless injector according to the present embodiment can include a material containing a component expected to have effects in the intradermal space or a component expected to exhibit a predetermined function in the intradermal space. Thus, as long as ejection with the energy for pressurizing described above can be achieved, a physical mode of the injection objective substance may be present in a state of being dissolved in liquid, or may be in a state of simply being mixed without being dissolved in liquid. As one example, the predetermined substance to be sent includes vaccine for intensifying an antibody, a protein for cosmetic enhancement, a cultured cell for hair regeneration, and the like, and is included in a liquid medium in an ejectable manner. The injection objective substance is formed in this way. Note that, the medium described above is preferably a medium that does not hinder the above-described effects and function of the predetermined substance in a state of being intradermally injected. As another method, the medium described above may be a medium that exhibits the above-described effects and function by acting together with the predetermined substance in the state of being intradermally injected.

The ejection pressure of the injection objective substance that is formed by pressurizing by the pressurizing unit is raised to a first peak pressure after the pressurizing is started, is lowered to a pressure lower than the first peak pressure afterward, and is raised to a second peak pressure again.

The first peak pressure is a characteristic pressure mainly required when the ejected injection objective substance perforates and penetrates the target region and enters the inside thereof at the initial stage. The second peak pressure that emerges later is a characteristic pressure required when most of the injection objective substance is delivered into the intradermal space.

Note that, the ejection pressure is defined as a pressure of the injection objective substance that is ejected through the ejection port, and is a pressure applied to the injection objective substance immediately after ejection through the ejection port, that is, in the vicinity of the ejection port, and a pressure for the injection objective substance to be ejected through the ejection port.

An area of the ejection port of the needleless injector according to the present embodiment may be an area of an ejection port of a normal needleless injector. For example, the area is from 0.0046 mm² to 0.0095 mm². Further, the area may be from 0.0028 mm² to 0.023 mm².

In addition, a diameter of a nozzle of the needleless injector according to the present embodiment may be a diameter of a nozzle of a normal needleless injector. For example, the diameter is from 0.09 mm to 0.11 mm. Further, the diameter may be from 0.06 mm to 0.17 mm.

In a needleless injector in which an ejection pressure that changes through the two peak pressures is provided, a magnitude of the energy n1 × T1 (mol · K) (that has a relationship with a magnitude of the first peak pressure) and a magnitude of the energy n2 × T2 (mol · K) (that has a relationship with a magnitude of the second peak pressure) dominantly determine the arrival of the injection objective substance into the intradermal space.

The needleless injector according to the present embodiment includes an igniter including an ignition agent and a gas generating agent that is disposed in a combustion chamber into which a combustion product (a flame or combustion gas) generated by combustion of the ignition agent flows and that is ignited by the combustion product of the ignition agent and thus generates a combustion product (a flame or combustion gas).

The ignition agent is preferably an explosive containing zirconium and potassium perchlorate (ZPP), an explosive containing zirconium, tungsten and potassium perchlorate (ZWPP), an explosive containing titanium hydride and potassium perchlorate (THPP), an explosive containing titanium and potassium perchlorate (TiPP), an explosive that can be used as an ignition agent for a gas generator, or an explosive containing a combination of a plurality of explosives among these explosives.

Further, the gas generating agent is formed in a manner that a combustion rate thereof is lower than a combustion rate of the ignition agent, and a combustion duration thereof is longer than a combustion duration of the ignition agent. The combustion rate is represented by a distance required from starting combustion from one end of the gas generating agent having a predetermined shape under a predetermined pressure to causing the combustion to reach the opposite end (a predetermined combustion distance) and a time period required thereto, and the combustion duration is represented by a time period from a combustion start time to a combustion end time.

Examples of the gas generating agent include a gas generating agent containing, as a main component, nitrocellulose that is ignited by a combustion product generated by combustion of the ignition agent and generates gas. Further, various types of gas generating agents used in a single-base smokeless explosive, a gas generator for an air bag, and a gas generator for a seat belt pretensioner may be used.

Specific examples thereof include an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), an explosive containing aluminum and iron oxide (AFO), single-base smokeless explosives listed as M1, M6, and M10 in Interior Ballistics of Guns, Progress in Astronautics and Aeronautics, Martin Summerfield Series Editor-in-Chief, Volume 66, a double-base explosive listed as M2, triple-base explosives listed as M30 and M31, and a mixed explosive containing a combination of a plurality of explosives among these explosives.

Further, the pressurizing unit causes an ejection pressure of the injection objective substance to reach the first peak pressure by a pressure of a combustion gas of the ignition agent by activating the igniter, and causes the ejection pressure of the injection objective substance to reach the second peak pressure due to application of a pressure of combustion gas of the gas generating agent that is combusted subsequently to the ignition agent. In this manner, the ignition agent and the gas generating agent are combusted in a linked manner, and thus the injection objective substance can be pressurized by the pressurizing unit as described above.

As a feature of the ignition agent, after the ejection pressure reaches the first peak pressure, the ejection pressure rapidly decreases. After that, the gas generating agent is combusted, and the ejection pressure reaches the second peak pressure relatively gradually. The injection objective substance can suitably reach the intradermal space by performing the pressure transition formation of the ejection pressure at the initial stage mainly with the ignition agent and performing the transition formation of the ejection pressure afterward with the gas generating agent as described above.

In the needleless injector according to the present embodiment, as described above, the igniter is activated to cause an ejection pressure of the injection objective substance to reach the first peak pressure by a pressure of combustion gas of the ignition agent. The first peak pressure is mainly a characteristic pressure required when the initially ejected injection objective substance perforates and penetrates the target region and enters the inside thereof. Thus, a value of the energy n1 × T1 (mol · K) related to the first peak pressure is set within a predetermined range, and thus, the injection objective substance is intradermally delivered.

In addition, in the needleless injector according to the present embodiment, as described above, a pressure of combustion gas of the ignition agent is added with a pressure of combustion gas of the gas generating agent that is combusted subsequently to the ignition agent, and thus, the ejection pressure of the injection objective substance reaches the second peak pressure. The second peak pressure is a characteristic pressure required when most of the injection objective substance is intradermally delivered. Thus, a value of the energy n2 × T2 (mol · K) related to the second peak pressure is set within a predetermined range, and thus, the injection objective substance is intradermally delivered.

The sum of the energy n1 × T1 (mol · K) and the energy n2 × T2 (mol · K) is not particularly limited as long as the injection objective substance can be intradermally delivered, and is preferably from 4.6 mol · K to 11.8 mol · K. Additionally, for example, an upper limit or a lower limit may be set to a value selected from 4.8 mol · K, 5.0 mol · K, 5.2 mol · K, 5.4 mol · K, 5.6 mol · K, 5.8 mol · K, 5.9 mol · K, 6.0 mol · K, 6.1 mol · K, 6.2 mol · K, 6.3 mol · K, 6.4 mol · K, 6.6 mol · K, 6.7 mol · K, 6.8 mol · K, 6.9 mol · K, 7.0 mol · K, 7.1 mol · K, 7.2 mol · K, 7.4 mol · K, 7.5 mol · K, 7.6 mol · K, 7.7 mol · K, 7.8 mol · K, 7.9 mol · K, 8.0 mol · K, 8.2 mol · K, 8.3 mol · K, 8.4 mol · K, 8.5 mol · K, 8.6 mol · K, 8.8 mol · K, 8.9 mol · K, 9.0 mol · K, 9.1 mol · K, 9.2 mol · K, 9.3 mol · K, 9.4 mol · K, 9.5 mol · K, 9.6 mol · K, 9.7 mol · K, 9.8 mol · K, 9.9 mol · K, 10.0 mol · K, 10.1 mol · K, 10.2 mol · K, 10.3 mol · K, 10.4 mol · K, 10.5 mol · K, 10.6 mol · K, 10.7 mol · K, 10.8 mol · K, 10.9 mol · K, 11.0 mol · K, 11.1 mol · K, 11.2 mol · K, 11.3 mol · K, 11.4 mol · K, or 11.6 mol · K.

There is the following relationship between the energy n1 × T1 (mol · K) and the energy n2 × T2 (mol · K) as the energy required to intradermally deliver the injection objective substance.

For example, contribution of the gas temperature to an energy of a combustion gas generated by combustion of the ignition agent is large, and contribution of the number of moles of the gas is small. That is, since the gas temperature rapidly increases in combustion and the gas temperature easily decreases after the combustion, an instantaneous (non-continuous) energy is supplied from the ignition agent. Thus, the energy of the combustion gas generated by the combustion of the ignition agent is used exclusively for perforating the skin and partially supplying the injection objective substance to the inside of the skin. On the other hand, the energy of the combustion gas generated by the combustion of the gas generating agent is more contributed by the number of moles of the gas, and is less affected by a decrease in temperature. Further, it is considered that the injection objective substance is delivered in a diffused manner within a wide range in the intradermal space by continuously supplying the energy. For this reason, even when the energy of the combustion gas generated in combustion of the ignition agent is relatively small and the injection objective substance is not sufficiently supplied to the inside of the skin at the initial stage, it is presumed that the energy of the combustion gas generated by the gas generating agent may complementarily act as long as the perforation is formed. That is, it is presumed that the energy of the combustion gas of the gas generating agent acts on wide diffusion of the injection objective substance ejected at the initial stage at a predetermined depth corresponding to the intradermal space.

To be specific, when the energy n1 × T1 (mol · K) is within the range from 1.0 mol · K to 2.7 mol · K and the energy n2 × T2 (mol · K) exceeds 9.2 mol · K, it is estimated that the injection objective substance eventually passes through the intradermal space and is delivered to the dermis. Thus, when the energy n1 × T1 (mol · K) is within the range from 1.0 mol · K ton 2.7 mol · K, the energy n2 × T2 (mol · K) is preferably defined to be equal to or less than 9.2 mol · K.

As described above, the energy n1 × T1 (mol · K) and the energy n2 × T2 (mol · K) have a relationship in which the injection objective substance is intradermally delivered in cooperation with each other.

Further, when the energy n2 × T2 (mol · K) is less than 3.7 mol · K, even when the energy n1 × T1 (mol · K) is within the range from 1.0 mol · K to 2.7 mol · K, it is presumed that the injection objective substance cannot be made to reach the intradermal space by the energy n1 × T1 (mol · K) and the injection objective substance cannot be made to reach the intradermal space by the energy n2 × T2 (mol · K), or it is presumed that the injection objective substance is pushed back from the intradermal space to the skin surface or the horny layer by restoration (elasticity) of the skin and the injection objective substance cannot be made to reach the intradermal space again even by the energy n2 × T2 (mol · K) while the ejection pressure decreases to a pressure lower than the first peak pressure after reaching the first peak pressure even when the injection objective substance can reach the intradermal space by the energy n1 × T1 (mol · K). Thus, when the energy n1 × T1 (mol · K) is within the range from 1.0 mol · K to 2.7 mol · K, the energy n2 × T2 (mol · K) is preferably defined to be equal to or more than 3.7 mol · K.

Furthermore, when the energy n1 × T1 (mol · K) is less than 1.0 mol · K, even when the energy n2 × T2 (mol · K) is within the range from 3.7 mol · K to 9.2 mol · K, it is presumed that the injection objective substance cannot be intradermally delivered because the injection objective substance cannot perforate and penetrate the target region and enter the inside of the target region by the energy n1 × T1 (mol · K), and the injection objective substance cannot perforate and penetrate the target region and enter the inside of the target region even by the energy n2 × T2 (mol · K). Thus, when the energy n2 × T2 (mol · K) is within the range from 3.7 mol · K to 9.2 mol · K, the energy n1 × T1 (mol · K) is preferably defined to be equal to or more than 1.0 mol · K.

Furthermore, when the energy n1 × T1 (mol · K) exceeds 2.7 mol · K, even when the energy n2 × T2 (mol · K) is within the range from 3.7 mol · K to 9.2 mol . K, it is presumed that the injection objective substance is delivered to a deep portion of the skin by the first peak pressure, and eventually the injection objective substance is diffused at a deeper portion than the intradermal space. Thus, when the energy n2 × T2 (mol · K) is within the range from 3.7 mol · K to 9.2 mol · K, the energy n1 × T1 (mol · K) is preferably defined to be equal to or less than 2.7 mol · K.

Additionally, the energy n1 × T1 (mol · K) may have an upper limit or a lower limit having a value selected from 1.1 mol · K, 1.3 mol · K, 1.5 mol · K, 1.7 mol · K, 1.9 mol · K, 2.1 mol · K, 2.3 mol · K, or 2.5 mol · K.

Additionally, the energy n2 × T2 (mol · K) may have an upper limit or a lower limit having a value selected from 4.3 mol · K, 4.9 mol · K, 5.5 mol · K, 6.1 mol · K, 6.7 mol · K, 7.3 mol · K, 7.9 mol · K, or 8.5 mol · K.

The energy n1 × T1 (mol · K) can be converted with respect to an amount of the ignition agent by n1 and T1 that are obtained by simulation using NEWPEP disclosed in JP 2010-269969 A and JP 2001-515009 T.

Further, the energy n2 × T2 (mol · K) can be converted with respect to an amount of the gas generating agent based on information disclosed in "Explosive Ammunition Technology Handbook, revised edition in 2012, published by Defense Technology Foundation" and "Interior Ballistics of Guns, Progress in Astronautics and Aeronautics, Martin Summerfield Series Editor-in-Chief, Volume 66".

Note that the expression "the injection objective substance has reached the intradermal space" refers to, for example, as in an example which will be described below, a case where the injection objective substance is ejected toward the skin surface by the number of samples N = 5 or more and a percentage of the number of times that the injection objective substance reaches the intradermal space, with respect to the number of times of ejection under the condition is equal to or larger than 60%, and the percentage is preferably large, and is equal to or larger than 65%, 70%, 75%, 80%, 85%, 90%, 95%, or 100% in an order being gradually preferable.

With reference to the drawings, a needleless injector 1 according to an embodiment of the present disclosure (herein, simply referred to as an "injector") will be described below. The injector 1 is a needleless injector that ejects an injection solution that corresponds to the injection objective substance in the present application, to a target region by use of a combustion energy of an explosive, that is, a device that intradermally injects the injection solution without using an injection needle. The injector 1 will be described below.

Note that, configurations of the following embodiment are provided as examples, and the invention of the present application is not limited to the configurations of the embodiment. Note that, in the present example, as terms indicating a relative positional relationship in a longitudinal direction of the injector 1, a "distal end side" and a "base end side" are used. The "distal end side" indicates a side close to the distal end of the injector 1 to be described later, that is, a position close to an ejection port 31a, and the "base end side" indicates a direction opposite to the "distal end side" in the longitudinal direction of the injector 1, that is, a direction toward a side of a drive unit 70.

### <Configuration of Injector 1>

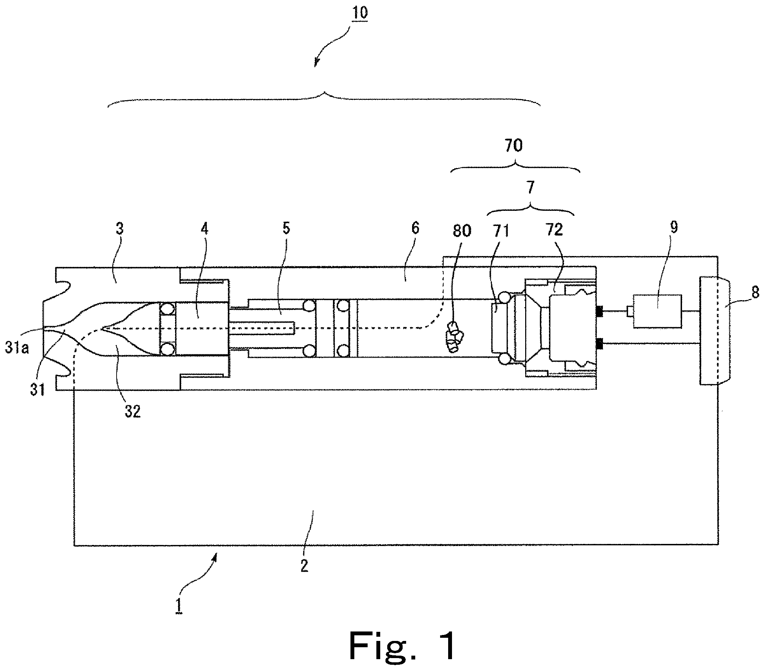

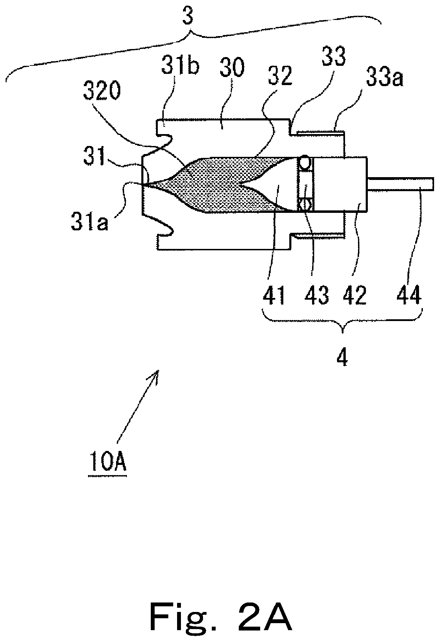

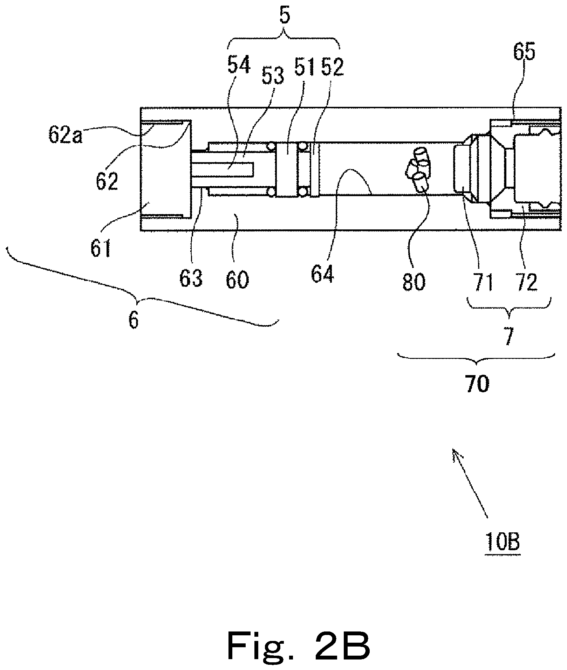

Here, FIG. 1 is a view illustrating a schematic configuration of the injector 1 and is also a cross-sectional view of the injector 1 taken along the longitudinal direction. In the injector 1, a device assembly 10 that is obtained by integrally assembling a sub-assembly 10A(see FIG. 2A to be described later) configured by a syringe unit 3 and a plunger 4 to be described later with a sub-assembly 10B (see FIG. 2B to be described later) configured by an injector main body 6, a piston 5, and the drive unit 70 is attached to a housing 2. Note that, in the following description in the present disclosure, the injection solution caused to reach the intradermal space by the injector 1 is formed of a liquid medium including a predetermined substance that exerts an effect or a function expected to the intradermal space. In the injection solution, the predetermined substance may be in a state of being dissolved in liquid being a medium, or may be in a state of being simply mixed instead of being dissolved.

Examples of the predetermined substance contained in the injection solution include biomolecules that can be intradermally injected. The biomolecules are not particularly limited as long as the biomolecules have physiological activity in the intradermal space when the biomolecules are intradermally injected. In addition, the biomolecules may be a natural product or an artificially synthesized product. Examples thereof include: a nucleic acid or a derivative thereof; a nucleoside, a nucleotide, or a derivative thereof; an amino acid, a peptide, a protein, or a derivative thereof; a lipid or a derivative thereof; a metal ion; a low molecular weight compound or a derivative thereof; an antibiotic; a vitamin or a derivative thereof; and the like. As long as it is a nucleic acid, it may be DNA or RNA, which may contain a gene or may be in the form of a vaccine. Further, examples of the biomolecules include low molecular drugs, inorganic substances such as metal particles for thermotherapy and radiotherapy, and substances having various pharmacological and therapeutic effects including a carrier, and the like. Further, the liquid being the medium of the injection solution is only required to be a substance suitable for causing the biomolecules to reach the intradermal space, and may be aqueous or oleaginous, which is not limited. Further, viscosity of the liquid being the medium is not particularly limited as long as the predetermined substance can be ejected by the injector 1.

The device assembly 10 is freely attachable to and detachable from the housing 2. A filling chamber 32 (see FIG. 2A) formed between the syringe unit 3 and the plunger 4 that are included in the device assembly 10 is filled with an injection solution. Then, the device assembly 10 can be configured as a unit that is replaced each time the injection solution is ejected, that is, as a cartridge. On the other hand, the housing 2 is configured as a control unit that performs ejection control of the injection solution by the device assembly 10, and a battery 9 that supplies power to an igniter 71 included in the drive unit 70 of the device assembly 10 is included on the housing 2 side. A user performs an operation of pressing down a button 8 provided to the housing 2, and thus the power supply from the battery 9 is performed between an electrode on the housing 2 side and an electrode on a side of an initiator 7 of the drive unit 70 of the device assembly 10 through a wiring line. Note that, the electrode on the housing 2 side and the electrode on the initiator 7 side of the drive unit 70 of the device assembly 10 are designed in shapes and at positions in a manner that both the electrodes are automatically held in contact with each other when the device assembly 10 is attached to the housing 2. Further, the housing 2 is a control unit that can be repeatedly used as long as power that can be supplied to the initiator 7 of the drive unit 70 is left in the battery 9. Further, in the housing 2, in a case where power of the battery 9 is exhausted, the housing 2 may be continuously used by replacing only the battery 9, or the battery 9 may be repeatedly used by charging.

Here, with reference to FIGS. 2A and 2B, configurations of the sub-assemblies 10A and 10B, and detailed configurations of the syringe unit 3, the plunger 4, the piston 5, the injector main body 6, and the drive unit 70 that are included in both the sub-assemblies will be described. The syringe unit 3 includes a nozzle unit 31 including the filling chamber 32 being a space capable of storing the injection solution. The plunger 4 is disposed in the sub-assembly 10A in a manner slidable in the filling chamber 32.

For a body 30 of the syringe unit 3, for example, nylon 6-12, polyarylate, polybutylene terephthalate, polyphenylene sulphide, a liquid crystal polymer, or the like, which are publicly known, may be used. Further, filler such as glass fibers and glass filler may be contained in those resins. 20 to 80 mass% of glass fibers may be contained in polybutylene terephthalate, 20 to 80 mass% of glass fibers may be contained in polyphenylene sulphide, or 20 to 80 mass% of minerals may be contained in a liquid crystal polymer.

Further, in the filling chamber 32 formed inside the body 30, the plunger 4 is disposed in a manner slidable in the nozzle unit 31 direction (the distal end side direction). A space formed between the plunger 4 and the body 30 of the syringe unit 3 is a space in which an injection solution 320 is enclosed. In the present embodiment, the filling chamber 32 in which the injection solution 320 is enclosed corresponds to the "enclosing unit". Herein, the plunger 4 slides in the filling chamber 32. Then the injection solution 320 stored in the filling chamber 32 is pressed, and is ejected through the ejection port 31a provided on the distal end side of the nozzle unit 31. Thus, the plunger 4 is formed of a material that smoothly slides in the filling chamber 32 and prevents the injection solution 320 from leaking from the plunger 4 side. Specific examples of the material of the plunger 4 include butyl rubber and silicone rubber. Further, there may be exemplified a styrene-based elastomer or a hydrogenated styrene-based elastomer, or a substance obtained by mixing a styrene-based elastomer or a hydrogenated styrene-based elastomer with polyolefin such as polyethylene, polypropylene, polybutene, and an α-olefin copolymer, oil such as liquid paraffin and process oil, or a powder inorganic substance such as talc, cast, and mica. Further, as the material of the plunger 4, there may be employed a polyvinyl chloride-based elastomer, an olefin-based elastomer, a polyester-based elastomer, a polyamide-based elastomer, a polyurethane-based elastomer, various rubber materials (particularly, a vulcanized material) such as natural rubber, isoprene rubber, chloroprene rubber, nitrile butadiene rubber, and styrene butadiene rubber, or a mixture thereof. Further, for the purpose of securing and adjusting slidability between the plunger 4 and the syringe unit 3, the surface of plunger 4 and the surface of the filling chamber 32 of the syringe unit 3 may be subjected to coating or surface finishing with various substances. Examples of the coating agent may include polytetrafluoroethylene (PTFE), silicone oil, diamond-like carbon, nanodiamond, and the like.

Here, as illustrated in FIG. 2A, the plunger 4 includes a head portion 41 and a barrel portion 42, which can be coupled by a neck portion 43 therebetween, the neck portion 43 having a diameter smaller than diameters of the head portion 41 and the barrel portion 42. The neck portion 43 has the small diameter in this manner, and thus, an accommodating space for an O-ring, which is a seal member, is formed. Note that a contour of the head portion 41 on the distal end side has a shape that substantially matches a contour of an inner wall surface of the nozzle unit 31. With this, when the plunger 4 slides to the nozzle unit 31 side at the time of ejection of the injection solution and reaches the deepest position located deepest in the filling chamber 32, the space formed between the plunger 4 and the inner wall surface of the nozzle unit 31 can be reduced as much as possible, and the injection solution 320 can be prevented from remaining in the filling chamber 32 and being wasted. However, the shape of the plunger 4 is not limited to a particular shape as long as desired effects can be obtained in the injector according to the present embodiment.

Furthermore, a rod portion 44, which extends from an end surface of the barrel portion 42 on the base end side in a direction further to the base end side, is provided on the plunger 4. The rod portion 44 has a diameter sufficiently smaller than a diameter of the barrel portion 42, which enables a user to grip the rod portion 44 in a manner movable in the filling chamber 32. Further, a length of the rod portion 44 is determined in a manner that even when the plunger 4 is at the deepest position in the filling chamber 32 of the syringe unit 3, the rod portion 44 protrudes from an end surface of the syringe unit 3 on the base end side, and a user can grip the rod portion 44.

Here, the description returns to the syringe unit 3. A flow path, which is provided to the nozzle unit 31 on the syringe unit 3 side, has an inner diameter formed smaller than an inner diameter of the filling chamber 32. With the configuration described above, the injection solution 320 pressurized to a high pressure is ejected to the outside through the ejection port 31a of the flow path. In view of this, a shield portion 31b having an annular shape and surrounding a periphery of the ejection port 31a is provided on the distal end side of the syringe unit 3, that is, in the vicinity of the nozzle unit 31. When the ejection port 31a is pressed against the target region such as the human skin, and the injection solution is ejected, the shield portion 31b can perform shielding and prevent the ejected injection solution from scattering in the periphery. Note that, the skin is pushed down to a certain degree when the ejection port is pressed against the skin surface, which can improve contact between the ejection port and the skin and prevent the injection solution from scattering. In view of this, as illustrated in FIG. 2A, the distal end of the nozzle unit 31 at which the ejection port 31a is positioned may slightly protrude from an end surface of the shield portion 31b in the ejection direction of the injection solution.

Further, a screw portion 33a that couples the injector main body 6 on the sub-assembly 10B side (to be described later) to the syringe unit 3 is formed at a neck portion 33 positioned on the base end side of the syringe unit 3. The diameter of the neck portion 33 is set to be smaller than the diameter of the body 30.

Next, with reference to FIG. 2B, the sub-assembly 10B including the piston 5, the injector main body 6, and the drive unit 70 will be described. Pressure is applied to the piston 5 by a combustion product generated by the igniter 71 of the drive unit 70, and the piston 5 is configured to slide inside a through-hole 64 formed inside a body 60 of the injector main body 6. Here, a coupling recess 61 is formed in the distal end side of the injector main body 6, with the through-hole 64 as a reference. The coupling recess 61 is a part that is coupled with the neck portion 33 of the syringe unit 3 described above, and a screw portion 62a, which is screwed together with the screw portion 33a provided on the neck portion 33, is formed on a side wall surface 62 of the coupling recess 61. Further, the through-hole 64 and the coupling recess 61 are communicated with each other by a communication portion 63, and the diameter of the communication portion 63 is set to be smaller than the diameter of the through-hole 64. Further, a drive unit recess 65 is formed on the base end side of the injector main body 6, with the through-hole 64 as a reference. The initiator 7 of the drive unit 70 is disposed in the drive unit recess 65.

Further, the piston 5 is formed of metal, and includes a first barrel portion 51 and a second barrel portion 52. The piston 5 is disposed in the through-hole 64, with the first barrel portion 51 being oriented to the coupling recess 61 side, and the second barrel portion 52 being oriented to the drive unit recess 65 side. The piston 5 slides inside the through-hole 64 in a state where the first barrel portion 51 and the second barrel portion 52 face an inner wall surface of the through-hole 64 of the injector main body 6. Note that, between the first barrel portion 51 and the second barrel portion 52, coupled is a coupling portion being narrower than the diameter of each of the barrel portions, and an O-ring or the like that improves adhesiveness with the inner wall surface of the through-hole 64 is disposed in a resultant space formed between both the barrel portions. Further, the piston 5 may be formed of a resin, and in this case, metal may also be used in parts for which heat resistance and pressure resistance are required.

Here, a pressing pillar portion 53 is provided on an end surface of the first barrel portion 51 on the distal end side. The diameter of the pressing pillar portion 53 is smaller than the diameter of the first barrel portion 51 and is smaller than the diameter of the communication portion 63 of the injector main body 6. An accommodation hole 54 is open in an end surface of the pressing pillar portion 53 on the distal end side. The diameter of the accommodation hole 54 is equal to or larger than the diameter of the rod portion 44, and the depth thereof is greater than the length of the rod portion 44. Thus, when the pressure is applied to the piston 5 by combustion gas generated by the igniter 71, the pressing pillar portion 53, via the end surface on the distal end side thereof, can transmit a combustion energy thereof to the end surface on the base end side of the barrel portion 42 of the plunger 4. Note that, the shape of the piston 5 is not limited to the shape described in FIG. 2B. In the present embodiment, the plunger 4 and the piston 5 constitute a "pressurizing unit".

Next, the drive unit 70 will be described. The drive unit 70 includes the initiator 7 and a gas generating agent 80, and the initiator 7 includes the igniter 71 and a body 72. The body 72 of the drive unit 70 is formed in a tubular shape, and the drive unit 70 includes the igniter 71 therein. The igniter 71 includes ignition agent and causes the ignition agent to combust and generates an energy for ejection. The initiator 7 of the drive unit 70 is disposed in the drive unit recess 65 as described above in a manner that the combustion energy generated by the igniter 71 is transmitted to the second barrel portion 52 of the piston 5. Specifically, the body 72 of the drive unit 70 may be obtained by fixing an injection molded resin to a metal collar. The injection molding can be performed by a known method. As the resin material for the body 72 of the drive unit 70, the same resin material as the body 30 of the syringe unit 3 may be employed.

Herein, an energy of a combustion gas in combustion of the ignition agent used in the igniter 71 is an energy for the injector 1 to perforate the target region by the injection solution and to inject the injection solution to the target region at a predetermined depth. Note that, examples of the preferred ignition agent include an explosive containing zirconium and potassium perchlorate (ZPP), an explosive containing zirconium, tungsten, and potassium perchlorate (ZWPP), an explosive containing titanium hydride and potassium perchlorate (THPP), an explosive containing titanium and potassium perchlorate (TiPP), and an explosive that can be used as an ignition agent of a gas generator, or an explosive containing a combination of a plurality of explosives among these explosives. An explosive other than these may be used as the ignition agent as long as appropriate ejection of the injection solution can be performed.

Further, in the injector 1, in addition to the above-mentioned ignition agent, the gas generating agent 80 is used for adjusting transition of the pressure to be applied to the injection solution through the piston 5. The gas generating agent 80 is ignited and combusted by a combustion product (flame or combustion gas) of the ignition agent in the igniter 71, and as a result, generates the combustion gas. In the present disclosure, a gas generating agent 80 constitutes a part of the drive unit 70, and is disposed at a location that can be exposed to the combustion product from the igniter 71, for example, as illustrated in FIGS. 1 and 2B. Further, as another method, the gas generating agent 80 may be disposed in the igniter 71 as disclosed in WO 01/031282, JP 2003-25950 A, and the like. As one example of the gas generating agent, there may be exemplified a single-base smokeless explosive containing 98 mass% of nitrocellulose, 1.0 mass% of diphenylamine, and 1.0 mass% of potassium sulfate. Further, various types of gas generating agents used in a gas generator for an air bag and a gas generator for a seat belt pretensioner may be used. A combustion completion time period of the gas generating agent can be changed by adjusting a dimension, a size, a shape, and particularly, a surface shape of the gas generating agent at the time of being disposed in the through-hole 64. With this, the transition of the pressure applied to the injection solution is adjusted, and an ejection pressure can be transitioned in a desired manner.

Note that, under a state in which the plunger 4 is inserted to the deepest position, the ejection port 31a is immersed in a container filled with the injection solution, and while maintaining this state, the plunger 4 is pulled back to the opening side of the filling chamber 32, that is, to the base end side of the syringe unit 3. In this manner, filling of the injection solution 320 in the sub-assembly 10A is performed. Note that, in this state, the plunger 4 is pulled back until the end surface of the barrel portion 42 of the plunger 4 on the base end side arrives at a position slightly protruding from the end surface of the syringe unit 3 on the base end side.

Further, in the sub-assembly 10B, the piston 5 is first inserted from the base end side of the injector main body 6 illustrated in FIG. 2B. At this time, the piston 5 is inserted into the through-hole 64, and the pressing pillar portion 53 is oriented toward the coupling recess 61 side. Then, an end surface on the distal end side of the piston 5, that is, an end surface on the distal end side of the pressing pillar portion 53 in which the accommodation hole 54 is open is positioned in a manner that the end surface protrudes by a predetermined amount from a bottom surface of the coupling recess 61 (a surface orthogonal to the side wall surface 62). When the piston 5 is positioned, a known technique, such as setting a mark for positioning in the through-hole 64 or using a jig for positioning may be employed as appropriate. Then, the gas generating agent 80 is disposed in the through-hole 64, and the initiator 7 of the drive unit 70 is attached to the drive unit recess 65. Note that a fixing force of the piston 5 in the through-hole 64 is set to an extent that the piston 5 can slide through the inside of the through-hole 64 in a sufficiently smooth manner as a result of the pressure received from the combustion product caused by the igniter 71 of the drive unit 70, and to an extent that, when the sub-assembly 10A is attached to the sub-assembly 10B, the piston 5 sufficiently resists a force received from the plunger 4, and the position of the piston 5 does not change.

The sub-assembly 10A having such a configuration is attached to the sub-assembly 10B by screwing together the screw portions 33a and 62a, and the device assembly 10 is thus formed. In this state, as the coupling of both the sub-assemblies progresses, the rod portion 44 of the plunger 4 advances and is accommodated in the accommodation hole 54 provided in the pressing pillar portion 53 of the piston 5. Finally, the end surface on the distal end side of the pressing pillar portion 53 is in a state of being in contact with the end surface on the base end side of the barrel portion 42 of the plunger 4. Note that the accommodation hole 54 has a size large enough to accommodate the rod portion 44, and thus, in this contact state, a deep inner wall surface of the accommodation hole 54 (in particular, a bottom surface of the accommodation hole 54) is not in contact with the end portion of the rod portion 44 on the base end side. Therefore, the rod portion 44 does not receive a load from the piston 5 side. Furthermore, as the screwing together progresses to a final position, the position of the piston 5 is fixed in the through-hole 64 with a sufficient frictional force, as described above, and thus, the plunger 4 is pressed by the pressing pillar portion 53 and advances to the ejection port 31a side, and the position of the plunger 4 is determined inside the syringe unit 3. Note that, a part of the injection solution 320 in accordance with an amount of the plunger 4 that is pushed out is discharged through the ejection port 31a.

When the plunger 4 is positioned at the final position in this way, the formation of the device assembly 10 is complete. In the device assembly 10, the piston 5 is positioned at a predetermined position with respect to the injector main body 6. With the piston 5 as a reference, the final position of the plunger 4 in the filling chamber 32 of the syringe unit 3 is automatically determined. The final position of the plunger 4 is a position uniquely determined in the device assembly 10, and hence an amount of the injection solution 320 that is finally stored in the filling chamber 32 can be a predetermined amount determined in advance.

Further, the device assembly 10 is attached to the housing 2, and a user presses down the button 8 under a state in which the ejection port 31a is held in contact with the target region. With this, the injection solution 320 is pressurized via the piston 5 and the plunger 4, and ejection is performed. Then, the injection solution 320 is ejected to the target region.

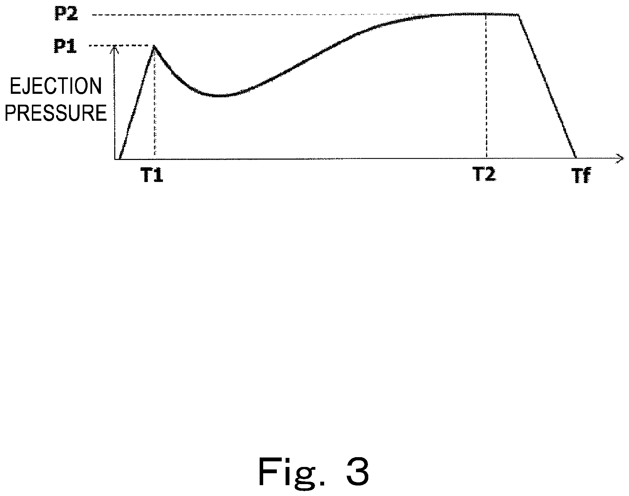

Herein, FIG. 3 illustrates transition of a pressure of the injection solution ejected through the ejection port 31a at the time of performing ejection of the injection solution by driving the drive unit 70 in the injector 1 (hereinafter, simply referred to as "ejection pressure"). In FIG. 3, a horizontal axis indicates elapsed time, and a vertical axis indicates an ejection pressure. Note that, the ejection pressure can be measured through use of a related-art technique. For example, an ejection force may be measured by a measurement method described in JP 2005-21640 A. That is, the ejection force is distributed and applied to a diaphragm of a load cell disposed downstream of the nozzle. Then, an output from the load cell is sampled by a data sampling device via a detection amplifier and stored as ejection force (N) per hour. The ejection force measured in this manner is divided by an area of the ejection port 31a of the injector 1, and thus the ejection pressure is calculated. Note that, in the example illustrated in FIG. 3, ZPP (including zirconium and potassium perchlorate) is employed as the ignition agent in the igniter 71 in the drive unit 70, and transition of the ejection pressure obtained by disposing the gas generating agent in the through-hole 64 is given.

Here, the ejection pressure transition illustrated in FIG. 3 is transition of the ejection pressure, with a timing at which the button 8 is pressed in the drive unit 70 as an initial point, during a time period from the combustion start to a timing at which the ejection pressure is substantially zero. Note that, the initial point does not indicate rise of the ejection pressure, but is shifted by a slight time period. This is because it takes a certain time period to combust the ignition agent, cause the piston 5 to advance by the combustion energy, pressurize the injection solution, and eject the injection solution through the ejection port 31a. Herein, in the injector 1, first, the ignition agent in the igniter 71 is combusted, and then the gas generating agent 80 is combusted as described above. With this, in the ejection pressure transition, two peak pressures P1 and P2 emerge substantially corresponding to the timings of the combustion. That is, the first peak pressure P1 that forms sudden pressure transition at the initial stage of the ejection pressure transition emerges due to the ignition agent that is combusted at a relatively high combustion rate. A timing at which the first peak pressure P1 emerges is referred to as a first timing T1. When the ignition agent is combusted, the combustion product generated herein is exposed to the gas generating agent, and hence combustion of the gas generating agent 80 is started. However, the above-mentioned ignition agent is in a gas form at a high temperature, but does not include a gaseous component at a room temperature. Thus, the ejection pressure is lowered immediately after the first timing T1. Meanwhile, the gas generating agent 80 is combusted at a slower combustion rate as compared to the ignition agent, and hence, even when combustion of the gas generating agent 80 is started, rise of the ejection pressure is relatively slow as compared to a process of reaching the first peak pressure. Thus, the second peak pressure P2 emerges at a second timing T2 delayed from the first timing T1. Note that, after the second peak pressure P2, the ejection pressure is lowered, and the ejection pressure is substantially zero at a pressurizing completion timing Tf.

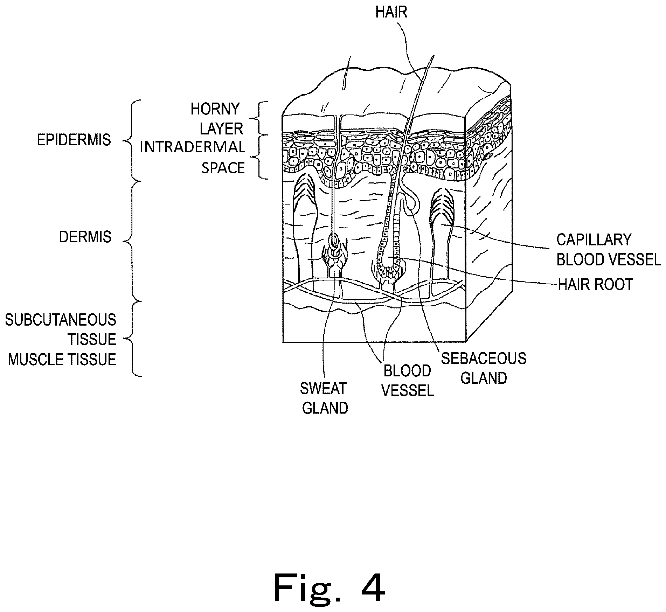

As described above, the injector 1 may be described as a device that pressurizes the injection solution and thus forms the ejection pressure illustrated in FIG. 3. The injection solution to which such ejection pressure is applied physically acts on the target region, perforates and penetrates the target region, and enters the inside. Thus, intradermal injection of the injection solution is achieved. Herein, examples of a target of the injector 1 include a skin structure body of an organism of a human, a farm animal, or the like. FIG. 4 schematically illustrates an anatomical structure of the human skin. The human skin has a layered structure including epidermis, dermis, a subcutaneous tissue, and a muscle tissue in the order from the skin surface side in a depth direction. Further, the epidermis can be sectionalized into a horny layer and an intradermal space in a layered manner. Main cells constituting tissues or the like and characteristics of the tissues differ among the respective layers of the skin structure body.

Specifically, the horny layer is mainly formed of a keratinocyte, and is positioned on the outermost surface side of the skin. Thus, the horny layer has a function of a so-called barrier layer. In general, the horny layer has a thickness of from substantially 0.01 mm to substantially 0.015 mm, and protects the surface of a human with the keratinocyte. Thus, relatively high strength is required for physically shielding the inside of the human body from an external environment to some extent. Further, the horny layer and the intradermal space form the epidermis, and the epidermis has a thickness of from substantially 0.1 mm to substantially 0.2 mm in general. Further, blood vessels and capillary blood vessels are spread in the dermis on the skin in a complex manner, and sweat glands for adjusting a body temperature, hair roots of body hair (including head hair), sebaceous glands associated therewith, and the like are also present in the dermis. Further, the dermis is a layer between an inside of a human body (the subcutaneous tissue and the muscle tissue) and the epidermis, and includes a fibroblast cell and a collagen cell. Thus, with regard to generation of wrinkles, loss of hair, and the like due to so-called lack of collagen or elastin, a state of the dermis is largely involved.

The target region to which the injection objective substance is ejected by the needleless injector according to the present embodiment and the intradermal space to which the injection objective substance is injected are preferably a target region of a mammal and the intradermal space to which the injection objective substance is injected, respectively. In addition, any of an in vitro system, an in vivo system, and an ex vivo system may be adoptable. That is, the target region and the intradermal space to which the injection objective substance is injected may be present in an individual (organism) or does not need to be present in an individual (organism). The latter includes, for example, a state of being removed or separated from an individual (organism). In addition, the latter may be an aspect excluding a target region in a state of being present in an individual (organism) and the intradermal space to which the injection objective substance is injected.

Examples of the mammal include humans and mammals other than humans. Examples of the human include a healthy person and a person (patient) suffering from a disease or the like. Examples of the mammals other than humans include pigs, cows, goats, sheep, monkeys, dogs, cats, rats, mice, hamsters, and guinea pigs.

In addition, the target region to which the injection objective substance is injected by the needleless injector according to the present embodiment and the intradermal space to which the injection objective substance is injected may be the skin surface and the intradermal space of the skin model, respectively. They may also be the surface and the intradermal space or the like derived from stem cells such as iPS cells (induced pluripotent stem cells). These may be present in an individual (organism) or does not need to be present in an individual (organism). The origin of the skin model and the stem cell is preferably a mammal, and the preferred form of the mammal is as described above.

Another embodiment of the present disclosure is a method for intradermally injecting an injection objective substance by using the needleless injector.

As the target region to which the injection objective substance is ejected by the needleless injector and the intradermal form to which the injection objective substance is injected, the above-described form is cited. Thus, the present embodiment may be, for example, a method of intradermally injecting an injection objective substance (however, excluding injection to the intradermal space existing on an individual (organism)) by using the needleless injector, a method of intradermally injecting an injection objective substance (however, excluding injection to the intradermal space of a human) by using the needleless injector, a method of intradermally injecting an injection objective substance to a mammal (however, excluding a human) by using the needleless injector, or the like.

### EXAMPLES

Examples will be described below, but none of the examples shall be construed as limiting.

Experimental conditions and experimental results will be described below for an injection experiment performed by using the injector 1 according to the present disclosure. The following experimental conditions were set for the purpose of achieving intradermal delivery of the injection solution to a pig. Since the skin of a pig is widely used in various tests as a model of the skin of a human, the results of this experiment can also be applied to a case where the skin of a human is a target.

### <Experimental Conditions>

### (About Injector 1)

As the ignition agent 22 of the initiator 20, a zirconium/potassium perchlorate (ZPP) mixture that generates gas having an energy value denoted in the vertical axis of Table 1 was used, and as the gas generating agent 30, a single-base smokeless explosive (M10) that generates gas having an energy value denoted in the horizontal axis of Table 1 was used. From Interior Ballistics of Guns, Progress in Astronautics and Aeronautics, Martin Summerfield Series Editor-in-Chief, Volume 66, a value under conditions that n1 of the ZPP was 0.94 (mol/100 g), and T1 = 4048 (K) was used, and the value was rounded to first decimal place. In addition, values of n2 = 4.07 (mol/100 g) and T2 = 3000 (K) in M10 by using NEWPEP were used, and the values were rounded off to first decimal place.

The numerical value in each cell of Table 1 is the sum of an energy value of gas generated by the ZPP mixture in combustion, which is denoted on the vertical axis, and an energy value of gas generated by the gas generating agent 30 in combustion, which is denoted on the horizontal axis. Note that, the sum is obtained by adding the energy value of the gas generated by the ZPP mixture in combustion, which is denoted on the vertical axis, and the energy value of the gas generated by the gas generating agent 30 in combustion, which is denoted on the horizontal axis, in consideration of each of the energy values to the second decimal place, and then rounding off the sum to the first decimal place. Thus, when the value is different from a numerical value obtained by simply adding the value on the vertical axis and the value on the horizontal axis in Table 1, the numerical value in each cell is treated as a normal sum as the sum.

Note that the diameter of the nozzle 4 is 0.1 mm, and one nozzle 4 is disposed in the syringe unit 3. In addition, composition percentages of the single-base smokeless explosive are as follows in M10 represented by, for example, Interior Ballistics of Guns, Progress in Astronautics and Aeronautics, Martin Summerfield Series Editor-in-Chief, Volume 66.
Nitrocellulose: 98.0 wt.%
Diphenylamine: 1.0 wt.%
Potassium sulfate: 1.0 wt.%
Graphite (in outer percentage): Trace

### (About Injection Target)

In this example, the skin surface of the abdomen of a pig was set as the target region. To be specific, the skin surface from the middle abdomen to the flank of a pig (a SPF piglet having a weight around 50 kg, LWD, female) was used as the injection target region. The pig was anesthetized immediately before the experiment and placed on a bed in a supine position to immobilize the four limbs. In this state, the nozzle unit 31 of the injector 1 was pressed against the skin as perpendicularly as possible, then the nozzle unit 31 was pushed into the skin surface under a load of 500 to 1500 g · f, and the switch was pressed to administer the injection solution. Note that when the skin of a killed pig is used as the injection target region, the pig skin may be stored in a refrigerated state or may be thawed after being stored in a frozen state, and the pressing load of the injector 1 is also 500 to 1500 g · f.

### (About Injection Solution)

A colored aqueous solution (methylene blue) was used, and an injection amount (ejection amount) was set to 100 µL. Thus, a state of diffusion of the injection solution after injection was easily grasped.

### <Experimental Results>

The experimental results are shown in Table 2. A cell in which "A" is denoted in Table 2 indicates a case where evaluation was performed with the number of samples N = 5 or more, and a percentage of the number of times that the injection objective substance has reached the intradermal space with respect to the number of times of injection under the experimental conditions was equal to or larger than 60%.

### [Table 1]

**Table 1**

| | | | | | | | | | | | |
|---|---|---|---|---|---|---|---|---|---|---|---|
| Energy of combustion gas of ignition agent (mol · K) | 2.7 | 6.3 | 6.9 | 7.5 | 8.2 | 8.8 | 9.4 | 10.0 | 10.6 | 11.2 | 11.8 |
| | 2.5 | 6.1 | 6.7 | 7.4 | 8.0 | 8.6 | 9.2 | 9.8 | 10.4 | 11.0 | 11.6 |
| | 2.3 | 5.9 | 6.6 | 7.2 | 7.8 | 8.4 | 9.0 | 9.6 | 10.2 | 10.8 | 11.4 |
| | 2.1 | 5.8 | 6.4 | 7.0 | 7.6 | 8.2 | 8.8 | 9.4 | 10.0 | 10.7 | 11.3 |
| | 1.9 | 5.6 | 6.2 | 6.8 | 7.4 | 8.0 | 8.6 | 9.2 | 9.8 | 10.4 | 11.1 |
| | 1.7 | 5.4 | 6.0 | 6.6 | 7.2 | 7.8 | 8.4 | 9.0 | 9.6 | 10.3 | 10.9 |
| | 1.5 | 5.2 | 5.8 | 6.4 | 7.0 | 7.6 | 8.2 | 8.8 | 9.5 | 10.1 | 10.7 |
| | 1.3 | 5.0 | 5.6 | 6.2 | 6.8 | 7.4 | 8.0 | 8.6 | 9.3 | 9.9 | 10.5 |
| | 1.1 | 4.8 | 5.4 | 6.0 | 6.6 | 7.2 | 7.9 | 8.5 | 9.1 | 9.7 | 10.3 |
| | 1.0 | 4.6 | 5.2 | 5.8 | 6.4 | 7.1 | 7.7 | 8.3 | 8.9 | 9.5 | 10.1 |
| | | 3.7 | 4.3 | 4.9 | 5.5 | 6.1 | 6.7 | 7.3 | 7.9 | 8.5 | 9.2 |
| | | Energy of combustion gas of gas generating agent (mol · K) | | | | | | | | | |

### [Table 2]

**Table 2**

| | | | | | | | | | | | |
|---|---|---|---|---|---|---|---|---|---|---|---|
| Energy of combustion gas of ignition agent (mol · K) | 2.7 | | A | A | | | | A | | | A |
| | 2.5 | A | A | A | | A | | A | A | A | |
| | 2.3 | | | | | A | A | | A | | |
| | 2.1 | A | | A | | A | | A | | | |
| | 1.9 | | | | | | A | | A | | |
| | 1.7 | | | A | | A | | A | A | A | A |
| | 1.5 | | A | A | | A | | A | A | A | |
| | 1.3 | | | | A | A | | A | A | | |
| | 1.1 | | | | A | | | | A | A | |
| | 1.0 | | | | A | A | | A | A | A | A |
| | | 3.7 | 4.3 | 4.9 | 5.5 | 6.1 | 6.7 | 7.3 | 7.9 | 8.5 | 9.2 |
| | | Energy of combustion gas of gas generating agent (mol · K) | | | | | | | | | |

### REFERENCE SIGNS LIST

1 Injector
2 Housing
3 Syringe unit
4 Plunger
5 Piston
6 Injector main body
7 Initiator
70 Drive unit
8 Button
9 Battery
10 Device assembly
10A, 10B Sub-assembly
30 Body
31 Nozzle unit
31a Ejection port
31b Shield portion
32 Filling chamber
33 Neck portion
33a Screw portion
41 Head portion
42 Barrel portion
43 Neck portion
44 Rod portion
51 First barrel portion
52 Second barrel portion
53 Pressing pillar portion
54 Accommodation hole
60 Body
61 Coupling recess
62 Side wall surface
62a Screw portion
63 Communication portion
64 Through-hole
65 Drive unit recess
71 Igniter
72 Body
80 Gas generating agent
320 Injection solution

## Claims

1. A device assembly for a needleless injector configured to intradermally inject an injection objective substance without using an injection needle, the device assembly comprising:
an enclosing unit configured to enclose the injection objective substance;
a drive unit including an igniter including an ignition agent and a gas generating agent disposed in a combustion chamber into which a combustion product generated by combustion of the ignition agent flows, the gas generating agent being configured to be ignited by the combustion product and thus to generate gas;
a pressurizing unit configured to pressurize, when the drive unit is driven, the injection objective substance enclosed in the enclosing unit; and
an ejection port through which the injection objective substance pressurized by the pressurizing unit is ejected to a target region,
wherein the gas generating agent is formed in a manner that a combustion rate of the gas generating agent is lower than a combustion rate of the ignition agent, and a combustion duration of the gas generating agent is longer than a combustion duration of the ignition agent,
the pressurizing unit is
configured to pressurize the injection objective substance in a manner that an ejection pressure of the injection objective substance increases to a first peak pressure after starting pressurization, then decreases to a pressure lower than the first peak pressure, and then increases again to a second peak pressure, the ejection pressure being defined as a pressure of the injection objective substance ejected from the ejection port,
the drive unit is
configured to activate the igniter and cause the ejection pressure of the injection objective substance to reach the first peak pressure by a pressure of a combustion gas of the ignition agent, and configured to cause the ejection pressure of the injection objective substance to reach the second peak pressure by application of the pressure of the combustion gas of the gas generating agent to be combusted subsequently to the ignition agent,
the ignition agent generates a combustion gas having a gas amount n1 (mol) and a temperature T1 (K) in combustion, and is defined in a manner that an energy value represented by n1 × T1 (mol · K) in the combustion gas of the ignition agent is from 1.0 mol · K to 2.7 mol · K, and
the gas generating agent generates a combustion gas having a gas amount n2 (mol) and a temperature T2 (K) in combustion, and is defined in a manner that an energy value represented by n2 × T2 (mol · K) in the combustion gas of the gas generating agent is from 3.7 mol · K to 9.2 mol · K.

2. The device assembly for the needleless injector according to claim 1,
wherein the ignition agent is any one of an explosive containing zirconium and potassium perchlorate, an explosive containing zirconium, tungsten, and potassium perchlorate, an explosive containing titanium hydride and potassium perchlorate, or an explosive containing titanium and potassium perchlorate, or an explosive containing a combination of a plurality of explosives among these explosives.

3. The device assembly for the needleless injector according to claim 1 or 2,
wherein the gas generating agent is a gas generating agent including any one of a single-base gas generating agent, a double-base gas generating agent, or a triple-base gas generating agent each containing nitrocellulose, or a gas generating agent including a combination of a plurality of gas generating agents among these gas generating agents.

4. A needleless injector comprising;
the device assembly for the needleless injector according to any one of claims 1 to 3; and
a control unit to which the device assembly for the needleless injector is attached, the control unit being configured to generate an activation signal for the igniter.

5. A method for intradermally injecting an injection objective substance by using the needleless injector according to claim 4.
